# FASCICULE DE BREVET EUROPEEN

(11) **EP 0 828 486 B1**
(45) Date de publication et mention de la délivrance du brevet: **18.09.2002**
(21) Numéro de dépôt: 96918727.7
(22) Date de dépôt: 29.05.1996
(51) Int. Cl.: A61K 31/40, A61K 31/425, A61P 25/32

(54) **UTILISATION DE DERIVES DE PYRROLIDINE AU TRAITEMENT DE L'ALCOOLISME**
VERWENDUNG VON PYRROLIDINDERIVATEN ZUR BEHANDLUNG DES ALKOHOLISMUS
USE OF PYRROLIDINE DERIVATIVES FOR TREATING ALCOHOLISM

(30) Priorité: 01.06.1995 FR 9506530
(43) Date de publication de la demande: 18.03.1998
(73) Titulaire: Aventis Pharma S.A., 92160 Antony (FR)
(72) Inventeur: IMPERATO, Assunta, 75014 Paris (FR)
(86) Numéro de dépôt international: FR9600801
(87) Numéro de publication internationale: WO96038139

(56) Documents cités:
- WO-A-93/01167
- WO-A-93/17011
- WO-A-94/15915

## Description

La présente invention concerne l'utilisation de dérivés de formule : 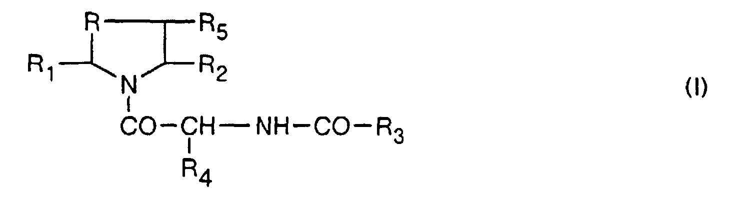 leurs racémiques et énantiomères lorsqu'il contiennent un ou plusieurs centres asymétriques et leurs sels au traitement de l'alcoolisme chronique ou des états dus à l'abus d'alcool et l'application de ces dérivés à la préparation de médicaments destinés au traitement de l'alcoolisme chronique ou des états dus à l'abus d'alcool.

Dans la formule (I),
- soit R représente un radical méthylène, éthylène, SO, SO₂, CHOH ou un atome de soufre, R₁ représente un radical pyridyle éventuellement substitué par un ou plusieurs radicaux alkyle, furyle éventuellement substitué par un ou plusieurs radicaux alkyle, thiènyle éventuellement substitué par un ou plusieurs radicaux alkyle, quinolyle éventuellement substitué par un ou plusieurs radicaux alkyle, naphtyle éventuellement substitué par un ou plusieurs radicaux alkyle, indolyle éventuellement substitué par un ou plusieurs radicaux alkyle ou phényle éventuellement substitué par un ou plusieurs substituants choisis parmi les atomes d'halogène et les radicaux alkyle, alcoxy, hydroxy, nitro, amino, monoalkylamino, dialkylamino, alcoxycarbonyle, -CO-NR₇R_{8,} -NH-CO-CH₃, trifluorométhyle ou trifluorométhoxy et R₅ représente un atome d'hydrogène,
- soit R représente un radical méthylène, R₁ représente un atome d'hydrogène et R₅ représente un radical phényle,
- soit R représente un radical CHR₆, R₁ et R₅ représentent chacun un atome d'hydrogène,
- R₂ représente un radical alcoxycarbonyle, cycloalkyloxycarbonyle, cycloalkylalkyloxycarbonyle, -CONR₉R₁₀ ou phényle éventuellement substitué par un ou plusieurs substituants choisis parmi les radicaux alkyle, alcoxy ou hydroxy,
- R₃ représente un radical phényle (éventuellement substitué par un ou plusieurs substituants choisis parmi les atomes d'halogène et les radicaux alkyle, alcoxy et alkylthio), naphtyle, indolyle, quinolyle ou phénylamino dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk'-COOX, -O-alk-COOX, -CH=CH-COOX, -CO-COOX, -alk-SO₃H sous forme de sel, -CH=CH-alk', -C(=NOH)-COOX, -S-alk-COOX, -O-CH₂-alk'-COOX, -CX=N-O-alk-COOX, -alk-N(OH)-CO-alk ou diméthyl-2,2 dioxo-4,6 dioxanne-1,3-yle-5,
- R₄ représente un atome d'hydrogène ou un radical alkyle,
- R₆ représente un radical phényle,
- R₇ représente un atome d'hydrogène ou un radical alkyle, phénylalkyle ou phényle éventuellement substitué par un ou plusieurs substituants choisis parmi les atomes d'halogène et les radicaux alkyle, alcoxy et alkylthio,
- R₈ représente un radical alkyle, phénylalkyle ou phényle éventuellement substitué par un ou plusieurs substituants choisis parmi les atomes d'halogène et les radicaux alkyle, alcoxy et alkylthio,
ou bien R₇ et R₈ forment avec l'atome d'azote auquel ils sont rattachés un hétérocycle mono ou polycyclique saturé ou insaturé contenant 4 à 9 atomes de carbone et un ou plusieurs hétéroatomes (O, N) et éventuellement substitué par un ou plusieurs radicaux alkyle,
- R₉ représente un atome d'hydrogène ou un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
- R₁₀ représente un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
ou bien R₉ et R₁₀ forment ensemble avec l'atome d'azote auquel ils sont rattachés un hétérocycle mono ou polycyclique saturé ou insaturé contenant 4 à 9 atomes de carbone et un ou plusieurs hétéroatomes (O, N, S) et éventuellement substitué par un ou plusieurs radicaux alkyle,
- X représente un atome d'hydrogène, un radical alkyle ou phénylalkyle,
- alk représente un radical alkyle ou alkylène,
- alk' représente un radical hydroxyalkyle, hydroxyalkylène, alkoxyalkyle ou alkoxyalkylène.

Dans les définitions qui précèdent et celles qui seront citées ci-après, sauf mention contraire, les radicaux alkyle, alkylène et alcoxy et les portions alkyle, alkylène et alcoxy contiennent 1 à 4 atomes de carbone en chaîne droite ou ramifiée, les radicaux ou portions acyle contiennent 2 à 4 atomes de carbone et les radicaux et portions cycloalkyle contiennent 3 à 6 atomes de carbone.

Lorsque R₇ et R₈ forment avec l'atome d'azote auquel ils sont rattachés un hétérocycle, celui-ci est de préférence un cycle pipéridino éventuellement substitué par un ou plusieurs radicaux alkyle ou un cycle tétrahydro-1,2,3,4 quinoléine.

Lorsque R₉ et R₁₀ forment avec l'atome d'azote auquel ils sont rattachés un hétérocycle, celui-ci est de préférence un cycle pipéridino, perhydroazépinyl-1, tétrahydro-1,2,3,6 pyridyl-1, tétrahydro-1,2,3,4 quinolyl-1, pyrrolidinyl-1, tétrahydro-1,2,3,4 isoquinolyl-2, thiomorpholino ou indolyl-1, ces cycles pouvant être éventuellement substitués par au moins un radical alkyle.

Les composés de formule (I) comportant un ou plusieurs centres asymétriques présentent des formes isomères. Les racémiques et les énantiomères de ces composés font également partie de l'invention.

Les composés de formule (I) peuvent éventuellement exister sous forme de sels d'addition avec un acide minéral ou organique.

Les composés de formule (I) comportant un reste carboxy, sulfo ou alk-SO₃H peuvent également éxister sous forme de sels métalliques ou de sels d'addition avec les bases azotées pharmaceutiquement acceptables.

Comme exemples de sels pharmaceutiquement acceptables, peuvent être cités les sels d'addition avec les acides minéraux ou organiques (tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate), les sels avec les métaux alcalins (sodium, potassium, lithium) ou avec les métaux alcalino-terreux (calcium, magnésium), le sel d'ammonium, les sels de bases azotées (éthanolamine, triméthylamine, méthylamine, benzylamine, N-benzyl-β-phénéthylamine, choline, arginine, leucine, lysine, N-méthyl glucamine).

Les produits de formule (I) et leurs sels peuvent être préparés dans les conditions décrites dans la demande internationale PCT WO 93/01167.

D'après la demande internationale PCT WO 93/01167, les composés de formule (I) présentent des propriétés pharmacologiques intéressantes. Ces composés possèdent une forte affinité pour les récepteurs de la cholécystokinine (CCK) et de la gastrine et sont donc utiles dans le traitement et la prévention des désordres liés à la CCK et à la gastrine au niveau du système nerveux et de l'appareil gastrointestinal.

C'est ainsi que selon la demande internationale PCT WO 93/01167, les composés peuvent être utilisés pour le traitement ou la prévention des psychoses, des troubles anxieux, de la maladie de Parkinson, de la diskinésie tardive, du syndrôme du colon irritable, de la pancréatite aiguë, des ulcères, des désordres de la motilité intestinale, de certaines tumeurs sensibles à la CCK et comme régulateur de l'appétit. Ces composés, qui ont également un effet de potentialisation sur l'activité analgésique des médicaments narcotiques et non narcotiques, peuvent avoir un effet analgésique propre. Par ailleurs, les composés qui ont une forte affinité pour les récepteurs CCK modifient les capacités de mémorisation, peuvent être efficaces dans les troubles de la mémoire.

Il a maintenant été trouvé que les composés de formule (I), leurs racémiques et énantiomères lorsqu'ils contiennent au moins un centre asymétrique et leurs sels sont particulièrement utiles pour le traitement de l'alcoolisme chronique ou des états résultant d'un abus d'alcool.

En se basant sur les travaux de H.H. Samson et R.A. Harris, Trends Pharmacol. Sci., 13, 206-211 (1992) et en effectuant les essais sur des rats entraînés à une forte consommation d'alcool, l'efficacité des composés selon l'invention peut être montrée en notant le comportement de rats vis-à-vis de la consommation de l'alcool. En effectuant des traitements répétés avec les produits selon l'invention à des doses comprises entre 5 et 25 mg/kg i.p. par jour pendant 14 jours la consommation d'alcool des animaux traités diminue des 2/3.

Chez le rat rendu alcool dépendant, les composés permettent une diminution de la consommation d'alcool pouvant atteindre plus de 40 % lorsque les composés selon l'invention sont administrés à des doses comprises entre 5 et 50 mg/kg par voie intrapéritonéale.

L'activité des produits peut aussi être mise en évidence chez les singes vivant dans les iles Caraïbes (Cercopithecus aethiops), dont certains consomment volontairement des boissons alcoolisées. Lorsque les composés selon l'invention sont administrés à des doses comprises entre 4 et 50 mg/kg par voie orale pendant 2 semaines à des singes habitués à consommer plus de 5 g d'éthanol par jour, la consommation diminue de 40 % la première semaine et de 30 % la seconde. Par ailleurs, les produits selon l'invention n'ont pas d'influence sur la consommation de nourriture puisque le poids moyen reste constant ainsi que sur la consommation d'eau.

Selon ces résultats, les produits selon l'invention permettent au patient de réduire de lui-même sa consommation d'alcool. Ces produits n'impliquent pas une privation d'alcool pour le patient pendant le traitement.

D'un intérêt particulier sont les composés de formule (I) pour lesquels R représente un radical méthylène, un atome de soufre ou un radical SO, R₁ représente un radical phényle éventuellement substitué, R₂ représente un radical phényle ou alcoxycarbonyle, R₄ et R₅ représente un atome d'hydrogène, R₃ représente un radical phénylamino dont le noyau phényle est substitué par un radical carboxy, -alk-COOH, -S-alk-COOH, hydroxyalkyle, alk'-COOH ou alkSO₃H, hydroxyiminoalkyle. Plus particulièrement intéressants sont les produits de formule (I) dans laquelle R₁ et R₂ sont en position cis l'un par rapport à l'autre.

D'un intérêt particulier sont les composés suivants :
- {[(hydroxy-1 éthyl-(RS))-3 phényl)-3 uréido]-2 acétyl}-1 phényl-5 prolinate de tert-butyle-(2RS,5SR),
- acide {{[tert-butoxycarbonyl-2 phényl-5 pyrrolidinyl-1 (2S,5R))-2 oxo-2 éthyl]-3 uréido}-3 phényl}-2 propionique,
- acide {{[(tert-butoxycarbonyl-2 phényl-5 pyrrolidinyl-1)-2 oxo-2 éthyl]-3 uréido}-3 phénylthio} acétique-(2RS,5SR),
- acide {[(tert-butoxycarbonyl-4 (fluoro-2 thiazolidinyl-3)-2 oxo-2 éthyl]-3 uréido}-3 phénylacétique-(2R,4R),
- acide {{[(tert-butoxycarbonyl-4 (fluoro-2 phényl)-2 thiazolidinyl-3-(2R,4R))-2 oxo-2 éthyl]-3 uréido}-3 phényl}-2 propionique,
- {{[(tert-butoxycarbonyl-4 phényl-2 thiazolidinyl-3-(2R,4R)))-2 oxo-2 éthyl]-3 uréido}-3 phényl}-1 éthanesulfonate de potassium-(RS),
- {{[(tert-butoxycarbonyl-2 phényl-5 pyrrolidinyl-1-(2S,5R))-2 oxo-2 éthyl]-3 uréido}-3 phényl}-1 éthanesulfonate de potassium-(RS),
- {{[(tert-butoxycarbonyl-2 phényl-5 pyrrolidinyl-1)-2 oxo-2 éthyl]-3 uréïdo}-3 phényl-1 méthanesulfonate de potassium-(2S,5R).
- acide {[tert-butoxycarbonyl-2 phényl-5 pyrrolidinyl-1)-2 oxo-2 éthyl]-3 uréido}-3 benzoïque-(2S,5R),
- acide {{[tert-butoxycarbonyl-2 (fluoro-2 phényl)-5 pyrrolidinyl-1]-2 oxo-2 éthyl}-3 uréido}-3 benzoïque-(2RS,5SR),
- acide {[(diphényl-2,5 pyrrolidinyl-1)-2 oxo-2 éthyl]-3 uréido}-3 benzoïque-(cis),
- acide {{[(hydroxy-2 phényl)-2 phényl-5 pyrrolidinyl-1]-2 oxo-2 uréido}-3 phénylacétique-(2RS,5SR),
- acide {[(tert-butoxycarbonyl-4 phényl-2 thiazolidinyl-3)-2 oxo-2 éthyl]-3 uréido}-3 phénylacétique-(2R,4R),
- acide {[(tert-butoxycarbonyl-4 phényl-2 thiazolidinyl-3)-2 oxo-2 éthyl]-3 uréido}-3 benzoïque-(2R,4R),
- acide {{[(tert-butoxycarbonyl-4 (fluoro-2 phényl)-2 oxyde-1 thiazolidinyl-3-(1RS,2R,4R))-2 oxo-2 éthyl}-3 uréido}-3 phényl}-2 propionique,
- acide {[(tert-butoxycarbonyl-4 (difluoro-2,3 phényl)-2 thiazolidinyl-3)-2 oxo-2 éthyl]-3 uréido}-3 phénylacétique-(2R,4R),
- {{[(hydroxyimino-1 éthyl)-3 phényl-(E)]-3 uréido}-2 acétyl}-1 phényl-5 prolinate de tert-butyle-(2RS,5SR)
et leurs sels.

Les médicaments selon l'invention sont constitués par un composé de formule (I) sous forme libre ou sous forme d'un sel d'addition avec un acide pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, iso-tonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les médicaments selon l'invention sont particulièrement utiles dans le traitement de l'alcoolisme chronique et des états dus à l'abus d'alcool.

Les doses dépendent de l'effet recherché, de la durée du traitement et de la voie d'administration utilisée; elles sont généralement comprises entre 0,05 g et 1 g par jour par voie orale pour un adulte avec des doses unitaires allant de 10 mg à 500 mg de substance active.

D'une façon générale, le médecin déterminera la posologie appropirée en fonction de l'âge, du poids et de tous les autres facteurs propres au sujet à traiter.

Les exemples suivants illustrent des médicaments selon l'invention :

### EXEMPLE A

On prépare, selon la technique habituelle, des gélules dosées à 50 mg de produit actif ayant la composition suivante :
- Composé de formule (I) 50 mg
- Cellulose 18 mg
- Lactose 55 mg
- Silice colloïdale 1 mg
- Carboxyméthylamidon sodique 10 mg
- Talc 10mg
- Stéarate de magnésium 1 mg

### EXEMPLE B

On prépare selon la technique habituelle de comprimés dosés à 50 mg de produit actif ayant la composition suivante :
- Composé de formule (I) 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante:
- Composé de formule (I) 10 mg
- Acide benzoïque 80 mg
- Alcool benzylique 0,06 cm³
- Benzoate de sodium 80 mg
- Ethanol à 95 % 0,4 cm³
- Hydroxyde de sodium 24 mg
- Propylène glycol 1,6 cm³
- Eau q.s.p. 4 cm3

## Revendications

1. Application des composés de formule : 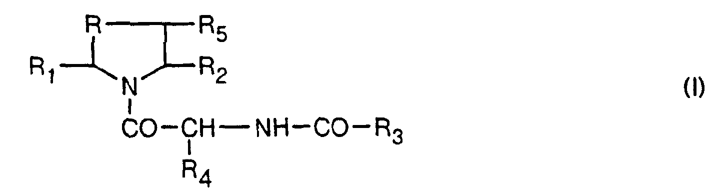 dans laquelle
- soit R représente un radical méthylène, éthylène, SO, SO₂, CHOH ou un atome de soufre, R₁ représente un radical pyridyle éventuellement substitué par un ou plusieurs radicaux alkyle, furyle éventuellement substitué par un ou plusieurs radicaux alkyle, thiènyle éventuellement substitué par un ou plusieurs radicaux alkyle, quinolyle éventuellement substitué par un ou plusieurs radicaux alkyle, naphtyle éventuellement substitué par un ou plusieurs radicaux alkyle, indolyle éventuellement substitué par un ou plusieurs radicaux alkyle ou phényle éventuellement substitué par un ou plusieurs substituants choisis parmi les atomes d'halogène et les radicaux alkyle, alcoxy, hydroxy, nitro, amino, monoalkylamino, dialkylamino, alcoxycarbonyle, -CO-NR₇R₈, -NH-CO-CH₃, trifluorométhyle ou trifluorométhoxy et R₅ représente un atome d'hydrogène,
- soit R représente un radical méthylène, R₁ représente un atome d'hydrogène et R₅ représente un radical phényle,
- soit R représente un radical CHR₆, R₁ et R₅ représentent chacun un atome d'hydrogène,
- R₂ représente un radical alcoxycarbonyle, cycloalkyloxycarbonyle, cycloalkylalkyloxycarbonyle, -CONR₉R₁₀ ou phényle éventuellement substitué par un ou plusieurs substituants choisis parmi les radicaux alkyle, alcoxy ou hydroxy,
- R₃ représente un radical phényle (éventuellement substitué par un ou plusieurs substituants choisis parmi les atomes d'halogène et les radicaux alkyle, alcoxy et alkylthio), naphtyle, indolyle, quinolyle ou phénylamino dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COO -alk-O-alk, -alk'-COOX, -O-alk-COOX, -CH=CH-COOX, -CO-COOX, -alk-SO₃H sous forme de sel, -CH=CH-alk', -C(=NOH)-COOX, -S-alk-COOX, -O-CH₂-alk'-COOX, -CX=N-O-alk-COOX, -alk-N(OH)-CO-alk ou diméthyl-2,2 dioxo-4,6 dioxanne-1,3-yle-5,
- R₄ représente un atome d'hydrogène ou un radical alkyle,
- R₆ représente un radical phényle,
- R₇ représente un atome d'hydrogène ou un radical alkyle, phénylalkyle ou phényle éventuellement substitué par un ou plusieurs substituants choisis parmi les atomes d'halogène et les radicaux alkyle, alcoxy et alkylthio,
- R₈ représente un radical alkyle, phénylalkyle ou phényle éventuellement substitué par un ou plusieurs substituants choisis parmi les atomes d'halogène et les radicaux alkyle, alcoxy et alkylthio,
ou bien R₇ et R₈ forment avec l'atome d'azote auquel ils sont rattachés un hétérocycle mono ou polycyclique saturé ou insaturé contenant 4 à 9 atomes de carbone et un ou plusieurs hétéroatomes (O, N) et éventuellement substitué par un ou plusieurs radicaux alkyle,
- R₉ représente un atome d'hydrogène ou un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
- R₁₀ représente un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
ou bien R₉ et R₁₀ forment ensemble avec l'atome d'azote auquel ils sont rattachés un hétérocycle mono ou polycyclique saturé ou insaturé contenant 4 à 9 atomes de carbone et un ou plusieurs hétéroatomes (O, N, S) et éventuellement substitué par un ou plusieurs radicaux alkyle,
- X représente un atome d'hydrogène, un radical alkyle ou phénylalkyle,
- alk représente un radical alkyle ou alkylène,
- alk' représente un radical hydroxyalkyle, hydroxyalkylène, alkoxyalkyle ou alkoxyalkylène, étant entendu que les radicaux alkyle, alkylène et alcoxy et les portions alkyle, alkylène et alcoxy contiennent 1 à 4 atomes de carbone en chaîne droite ou ramifiée, les radicaux et portions acyle contiennent 2 à 4 atomes de carbone et les radicaux et portions cycloalkyle contiennent 3 à 6 atomes de carbone, ainsi que leurs sels et leurs racémiques et énantiomères lorsqu'ils comportent au moins un centre asymétrique, à la préparation d'un médicament destiné au traitement de l'alcoolisme chronique ou des états dus à l'abus d'alcool.

2. Application d'un composé de formule (I) selon la revendication 1 pour lequel R représente un radical méthylène, un atome de soufre ou un radical SO, R₁ représente un radical phényle éventuellement substitué, R₂ représente un radical phényle ou alcoxycarbonyle, R₄ et R₅ représentent un atome d'hydrogène et R₃ représente un radical phénylamino dont le noyau phényle est substitué par un radical carboxy, -alk-COOH, -S-alk-COOH, hydroxyalkyle, alk'-COOH ou alk-SO₃H sous forme de sel, à la préparation d'un médicament destiné au traitement de l'alcoolisme chronique ou des états dus à l'abus d'alcool.

3. Application d'un composé de formule (I) selon la revendication 1 choisi parmi les composés suivants :
- {[(hydroxy-1 éthyl-(RS))-3 phényl)-3 uréido]-2 acétyl}-1 phényl-5 prolinate de tert-butyle-(2RS,5SR),
- acide {{[(tert-butoxycarbonyl-2 phényl-5 pyrrolidinyl-1 (2S,5R))-2 oxo-2 éthyl]-3 uréido}-3 phényl}-2 propionique,
- acide {{[(tert-butoxycarbonyl-2 phényl-5 pyrrolidinyl-1)-2 oxo-2 éthyl]-3 uréido}-3 phénylthio} acétique-(2RS,5SR),
- acide {[(tert-butoxycarbonyl-4 (fluoro-2 thiazolidinyl-3)-2 oxo-2 éthyl]-3 uréido}-3 phénylacétique-(2R,4R),
- acide {{[(tert-butoxycarbonyl-4 (fluoro-2 phényl)-2 thiazolidinyl-3-(2R,4R))-2 oxo-2 éthyl]-3 uréido}-3 phényl}-2 propionique,
- {{[(tert-butoxycarbonyl-4 phényl-2 thiazolidinyl-3-(2R,4R)))-2 oxo-2 éthyl]-3 uréido}-3 phényl}-1 éthanesulfonate de potassium-(RS),
- {{[(tert-butoxycarbonyl-2 phényl-5 pyrrolidinyl-1-(2S,5R))-2 oxo-2 éthyl]-3 uréido}-3 phényl}-1 éthanesulfonate de potassium-(RS),
- {{[(tert-butoxycarbonyl-2 phényl-5 pyrrolidinyl-1)-2 oxo-2 éthyl]-3 uréïdo}-3 phényl-1 méthanesulfonate de potassium-(2S,5R).
- acide {[tert-butoxycarbonyl-2 phényl-5 pyrrolidinyl-1)-2 oxo-2 éthyl]-3 uréido}-3 benzoïque-(2S,5R),
- acide {{[tert-butoxycarbonyl-2 (fluoro-2 phényl)-5 pyrrolidinyl-1]-2 oxo-2 éthyl}-3 uréido}-3 benzoïque-(2RS,5SR),
- acide {[(diphényl-2,5 pyrrolidinyl-1)-2 oxo-2 éthyl]-3 uréido}-3 benzoïque-(cis),
- acide {{[(hydroxy-2 phényl)-2 phényl-5 pyrrolidinyl-1]-2 oxo-2 uréido}-3 phénylacétique-(2RS,5SR),
- acide {[(tert-butoxycarbonyl-4 phényl-2 thiazolidinyl-3)-2 oxo-2 éthyl]-3 uréido}-3 phénytacétique-(2R,4R),
- acide {[(tert-butoxycarbonyl-4 phényl-2 thiazolidinyl-3)-2 oxo-2 éthyl]-3 uréido}-3 benzoïque-(2R,4R),
- acide {{[(tert-butoxycarbonyl-4 (fluoro-2 phényl)-2 oxyde-1 thiazolidinyl-3-(1RS,2R,4R))-2 oxo-2 éthyl}-3 uréido}-3 phényl}-2 propionique,
- acide {[(tert-butoxycarbonyl-4 (difluoro-2,3 phényl)-2 thiazolidinyl-3)-2 oxo-2 éthyl]-3 uréido}-3 phénylacétique-(2R,4R),
- {{[(hydroxyimino-1 éthyl)-3 phényl-(E)]-3 uréido}-2 acétyl}-1 phényl-5 prolinate de tert-butyle-(2RS,5SR),
leurs racémiques et énantiomères lorsqu'ils contiennent au moins un carbone asymétrique et leurs sels, à la préparation d'un médicament destiné au traitement de l'alcoolisme chronique ou des états dus à l'abus d'alcool.

4. Application de l'acide {{[(tert-butoxycarbonyl-4 (fluoro-2 phényl)-2 thiazolidinyl-3-(2R,4R))-2 oxo-2 éthyl]-3 uréido}-3 phényl}-2 propionique à la préparation d'un médicament destiné au traitement de l'alcoolisme chronique ou des états dus à l'abus d'alcool.

5. Application des composés de formule : 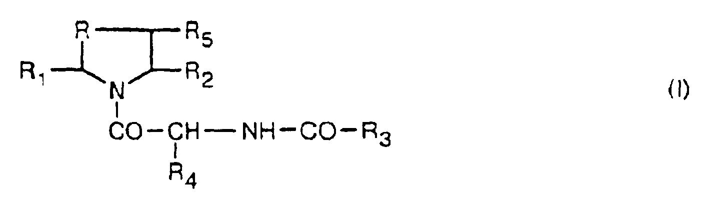 dans laquelle
- soit R représente un radical méthylène, éthylène, SO, SO₂, CHOH ou un atome de soufre, R₁ représente un radical pyridyle éventuellement substitué par un ou plusieurs radicaux alkyle, furyle éventuellement substitué par un ou plusieurs radicaux alkyle, thiènyle éventuellement substitué par un ou plusieurs radicaux alkyle, quino yle éventuellement substitué par un ou plusieurs radicaux alkyle, naphtyle éventuellement substitué par un ou plusieurs radicaux alkyle, indolyle éventuellement substitué par un ou plusieurs radicaux alkyle ou phényie éventuellement substitué par un ou plusieurs substituants choisis parmi les atomes d'halogène et les radicaux alkyle, alcoxy, hydroxy, nitro, amino, monoalkylamino, dialkylamino, alcoxycarbonyle, -CO-NR₇R₈,
- NH-CO-CH₃, trifluorométhyle ou trifluorométhoxy et R₅ représente un atome d'hydrogène,
- soit R représente un radical méthylène, R₁ représente un atome d'hydrogène et R₅ représente un radical phényle,
- soit R représente un radical CHR₆, R₁ et R₅ représentent chacun un atome d'hydrogène,
- R₂ représente un radical alcoxycarbonyle, cycloalkyloxycarbonyle, cycloalkylalkyloxycarbonyle, -CONR₉R₁₀ ou phényle éventuellement substitué par un ou plusieurs substituants choisis parmi les radicaux alkyle, alcoxy ou hydroxy,
- R₃ représente un radical phényle (éventuellement substitué par un ou plusieurs substituants choisis parmi les atomes d'halogène et les radicaux alkyle, alcoxy et alkylthio), naphtyle, indolyle, quinolyle ou phénylamino dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk'-COOX, -O-alk-COOX, -CH=CH-COOX, -CO-COOX, -alk-SO₃H sous forme de sel, -CH=CH-alk', -C(=NOH)-COOX, -S-alk-COOX, -O-CH₂-alk'-COOX, -CX=N-O-alk-COOX, -alk-N(OH)-CO-alk ou diméthyl-2,2 dioxo-4,6 dioxanne-1,3-yle-5,
- R₄ représente un atome d'hydrogène ou un radical alkyle,
- R₆ représente un radical phényle,
- R₇ représente un atome d'hydrogène ou un radical alkyle, phénylalkyle ou phényle éventuellement substitué par un ou plusieurs substituants choisis parmi les atomes d'halogène et les radicaux alkyle, alcoxy et alkylthio,
- R₈ représente un radical alkyle, phénylalkyle ou phényle éventuellement substitué par un ou plusieurs substituants choisis parmi les atomes d'halogène et les radicaux alkyle, alcoxy et alkylthio,
ou bien R₇ et R₈ forment avec l'atome d'azote auquel ils sont rattachés un hétérocycle mono ou polycyclique saturé ou insaturé contenant 4 à 9 atomes de carbone et un ou plusieurs hétéroatomes (O, N) et éventuellement substitué par un ou plusieurs radicaux alkyle,
- R₉ représente un atome d'hydrogène ou un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
- R₁₀ représente un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
ou bien R₉ et R₁₀ forment ensemble avec l'atome d'azote auquel ils sont rattachés un hétérocycle mono ou polycyclique saturé ou insaturé contenant 4 à 9 atomes de carbone et un ou plusieurs hétéroatomes (O, N, S) et éventuellement substitué par un ou plusieurs radicaux alkyle,
- X représente un atome d'hydrogène, un radical alkyle ou phénylalkyle,
- alk représente un radical alkyle ou alkylène,
- alk' représente un radical hydroxyalkyle, hydroxyalkylène, alkoxyalkyle ou alkoxyalkylène, étant entendu que les radicaux alkyle, alkylène et alcoxy et les portions alkyle, alkylène et alcoxy contiennent 1 à 4 atomes de carbone en chaîne droite ou ramifiée, les radicaux et portions acyle contiennent 2 à 4 atomes de carbone et les radicaux et portions cycloalkyle contiennent 3 à 6 atomes de carbone, ainsi que leurs sels et leurs racémiques et énantiomères lorsqu'ils comportent au moins un centre asymétrique, à la préparation d'un médicament permettant au patient de réduire de lui-même sa consommation d'alcool.

6. Application d'un composé de formule (I) selon la revendication 5 pour lequel R représente un radical méthylène, un atome de soufre ou un radical SO, R₁ représente un radical phényle éventuellement substitué, R₂ représente un radical phényle ou alcoxycarbonyle, R₄ et R₅ représentent un atome d'hydrogène et R₃ représente un radical phénylamino dont le noyau phényle est substitué par un radical carboxy, -alk-COOH, -S-alk-COOH, hydroxyalkyle, alk'-COO ou alk-SO₃H sous forme de sel, à la préparation d'un médicament permettant au patient de réduire de lui-même sa consommation d'alcool.

7. Application d'un composé de formule (I) selon la revendication 5 choisi parmi les composés suivants :
- {[(hydroxy-1 éthyl-(RS))-3 phényl)-3 uréido]-2 acétyl}-1 phényl-5 prolinate de tert-butyle-(2RS,5SR),
- acide {{[(tert-butoxycarbonyl-2 phényl-5 pyrrolidinyl-1 (2S,5R))-2 oxo-2 éthyl]-3 uréido}-3 phényl}-2 propionique,
- acide {{[(tert-butoxycarbonyl-2 phényl-5 pyrrolidinyl-1)-2 oxo-2 éthyl]-3 uréido}-3 phénylthio} acétique-(2RS,5SR),
- acide {[(tert-butoxycarbonyl-4 (fluoro-2 thiazolidinyl-3)-2 oxo-2 éthyl]-3 uréido}-3 phénylacétique-(2R,4R),
- acide {{[(tert-butoxycarbonyl-4 (fluoro-2 phényl)-2 thiazolidinyl-3-(2R,4R))-2 oxo-2 éthyl]-3 uréido}-3 phényl}-2 propionique,
- {{[(tert-butoxycarbonyl-4 phényl-2 thiazolidinyl-3-(2R,4R)))-2 oxo-2 éthyl]-3 uréido}-3 phényl}-1 éthanesulfonate de potassium-(RS),
- {{[(tert-butoxycarbonyl-2 phényl-5 pyrrolidinyl-1-(2S,5R))-2 oxo-2 éthyl]-3 uréido}-3 phényl}-1 éthanesulfonate de potassium-(RS),
- {{[(tert-butoxycarbonyl-2 phényl-5 pyrrolidinyl-1)-2 oxo-2 éthyl]-3 uréïdo}-3 phényl-1 méthanesulfonate de potassium-(2S,5R).
- acide {[tert-butoxycarbonyl-2 phényl-5 pyrrolidinyl-1)-2 oxo-2 éthyl]-3 uréido}-3 benzoïque-(2S,5R),
- acide {{[tert-butoxycarbonyl-2 (fluoro-2 phényl)-5 pyrrolidinyl-1]-2 oxo-2 éthyl}-3 uréido}-3 benzoïque-(2RS,5SR),
- acide {[(diphényl-2,5 pyrrolidinyl-1)-2 oxo-2 éthyl]-3 uréido}-3 benzoïque-(cis),
- acide {{[(hydroxy-2 phényl)-2 phényl-5 pyrrolidinyl-1]-2 oxo-2 uréido}-3 phénylacétique-(2RS,5SR),
- acide {[(tert-butoxycarbonyl-4 phényl-2 thiazolidinyl-3)-2 oxo-2 éthyl]-3 uréido}-3 phénylacétique-(2R,4R),
- acide {[(tert-butoxycarbonyl-4 phényl-2 thiazolidinyl-3)-2 oxo-2 éthyl]-3 uréido}-3 benzoïque-(2R,4R),
- acide {{[(tert-butoxycarbonyl-4 (fluoro-2 phényl)-2 oxyde-1 thiazolidinyl-3-(1RS,2R,4R))-2 oxo-2 éthyl}-3 uréido}-3 phényl}-2 propionique,
- acide {[(tert-butoxycarbonyl-4 (difluoro-2,3 phényl)-2 thiazolidinyl-3)-2 oxo-2 éthyl]-3 uréido}-3 phénylacétique-(2R,4R),
- {{[(hydroxyimino-1 éthyl)-3 phényl-(E)]-3 uréido}-2 acétyl}-1 phényl-5 prolinate de tert-butyle-(2RS,5SR),
leurs racémiques et énantiomères lorsqu'ils contiennent au moins un carbone asymétrique et leurs sels, à la préparation d'un médicament permettant au patient de réduire de lui-même sa consommation d'alcool.

8. Application de l'acide {{[(tert-butoxycarbonyl-4 (fluoro-2 phényl)-2 thiazolidinyl-3-(2R,4R))-2 oxo-2 éthyl]-3 uréido}-3 phényl}-2 propionique à la préparation d'un médicament permettant au patient de réduire de lui-même sa consommation d'alcool.

## Claims

1. Application of the compounds of formula: 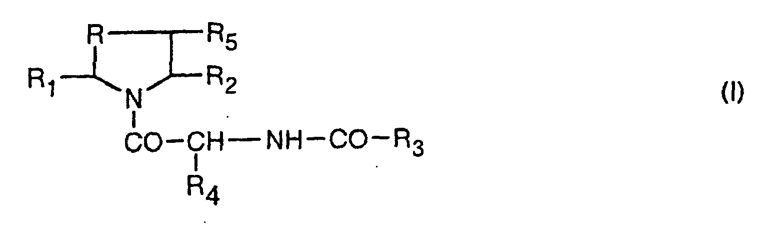 in which
- either R represents a methylene, ethylene, SO, SO₂, or CHOH radical or a sulphur atom, R₁ represents a pyridyl radical which is optionally substituted by one or more alkyl radicals, a furyl radical which is optionally substituted by one or more alkyl radicals, a thienyl radical which is optionally substituted by one or more alkyl radicals, a quinolyl radical which is optionally substituted by one or more alkyl radicals, a naphthyl radical which is optionally substituted by one or more alkyl radicals, an indolyl radical which is optionally substituted by one or more alkyl radicals or a phenyl radical which is optionally substituted by one or more substituents chosen from halogen atoms and alkyl, alkoxy, hydroxyl, nitro, amino, monoalkylamino, dialkylamino, alkoxycarbonyl, -CO-NR₇R₈, -NH-CO-CH₃, trifluoromethyl or trifluoromethoxy radicals and R₅ represents a hydrogen atom,
- or R represents a methylene radical, R₁ represents a hydrogen atom and R₅ represents a phenyl radical,
- or R represents a CHR₆ radical and R₁ and R₅ each represent a hydrogen atom,
- R₂ represents an alkoxycarbonyl, cycloalkyloxycarbonyl, cycloalkylalkyloxycarbonyl,
- CONR₉R₁₀ or phenyl radical which is optionally substituted by one or more substituents chosen from alkyl, alkoxy or hydroxyl radicals,
- R₃ represents a phenyl radical (optionally substituted by one or more substituents chosen from halogen atoms and alkyl, alkoxy and alkylthio radicals), a naphthyl radical, an indolyl radical, a quinolyl radical or a phenylamino radical in which the phenyl ring is optionally substituted by one or more substituents chosen from halogen atoms and alkyl, alkoxy, alkylthio, trifluoromethyl, carboxyl, alkoxycarbonyl, hydroxyl, nitro, amino, acyl, cyano, sulphamoyl, carbamoyl, hydroxyiminoalkyl, alkoxyiminoalkyl, hydroxyaminocarbonyl, alkoxyaminocarbonyl, tetrazol-5-yl, tetrazol-5-ylalkyl, trifluoromethylsulphonamido, alkylsulphinyl, mono- or polyhydroxyalkyl, sulpho, -alk-O-CO-alk, -alk-COOX, -alk-O-alk, -alk'-COOX, -O-alk-COOX, -CH=CH-COOX, -CO-COOX, -alk-SO₃H in the salt form, -CH=CH-alk', -C(=NOH)-COOX, -S-alk-COOX, -O-CH₂-alk'-COOX, -CX=N-O-alk-COOX, -alk-N(OH)-CO-alk or 2,2-dimethyl-4,6-dioxo-1,3-dioxan-5-yl radicals,
- R₄ represents a hydrogen atom or an alkyl radical,
- R₆ represents a phenyl radical,
- R₇ represents a hydrogen atom or an alkyl, phenylalkyl or phenyl radical which is optionally substituted by one or more substituents chosen from halogen atoms and alkyl, alkoxy and alkylthio radicals,
- R₈ represents an alkyl, phenylalkyl or phenyl radical which is optionally substituted by one or more substituents chosen from halogen atoms and alkyl, alkoxy and alkylthio radicals,
or else R₇ and R₈ form, with the nitrogen atom to which they are attached, a saturated or unsaturated, mono- or polycyclic heterocycle containing 4 to 9 carbon atoms and one or more heteroatoms (O, N) and which is optionally substituted by one or more alkyl radicals,
- R₉ represents a hydrogen atom or an alkyl, cycloalkylalkyl, cycloalkyl, phenylalkyl or phenyl radical which is optionally substituted by one or more substituents chosen from halogen atoms and alkyl, alkoxy and alkylthio radicals,
- R₁₀ represents an alkyl, cycloalkylalkyl, cycloalkyl, phenylalkyl or phenyl radical which is optionally substituted by one or more substituents chosen from halogen atoms and alkyl, alkoxy and alkylthio radicals,
or else R₉ and R₁₀ form, together with the nitrogen atom to which they are attached, a saturated or unsaturated, mono- or polycyclic heterocycle containing 4 to 9 carbon atoms and one or more heteroatoms (O, N, S) and which is optionally substituted by one or more alkyl radicals,
- X represents a hydrogen atom or an alkyl or phenylalkyl radical,
- alk represents an alkyl or alkylene radical,
- alk' represents a hydroxyalkyl, hydroxyalkylene, alkoxyalkyl or alkoxyalkylene radical, it being understood that the alkyl, alkylene and alkoxy radicals and the alkyl, alkylene and alkoxy portions contain 1 to 4 straight- or branched-chain carbon atoms, the acyl radicals and portions contain 2 to 4 carbon atoms and the cycloalkyl radicals and portions contain 3 to 6 carbon atoms, and their salts and their racemates and enantiomers, when they contain at least one asymmetric centre, to the preparation of a medicament intended for the treatment of chronic alcoholism or of conditions due to alcohol abuse.

2. Application of a compound of formula (I) according to Claim 1 for which R represents a methylene radical, a sulphur atom or an SO radical, R₁ represents an optionally substituted phenyl radical, R₂ represents a phenyl or alkoxycarbonyl radical, R₄ and R₅ represent a hydrogen atom and R₃ represents a phenylamino radical in which the phenyl ring is substituted by a carboxyl, -alk-COOH, -S-alk-COOH, hydroxyalkyl, alk'-COOH or alk-SO₃H radical in the salt form to the preparation of a medicament intended for the 'treatment of chronic alcoholism or of conditions due to alcohol abuse.

3. Application of a compound of formula (I) according to Claim 1 chosen from the following compounds:
- tert-butyl (2RS,5SR)-1-{2-[3-(3-(RS)-1-hydroxyethyl)-phenyl)ureido]acetyl}-5-phenylprolinate,
- 2-{3-{3-[2-((2S,5R)-2-tert-butoxycarbonyl-5-phenyl-1-pyrrolidinyl)-2-oxoethyl]ureido}phenyl}propionic acid
- (2RS,5SR)-{3-{3-[2-(2-tert-butoxycarbonyl-5-phenyl-1-pyrrolidinyl)-2-oxoethyl]ureido}phenylthio}acetic acid,
- (2R,4R)-3-{3-[2-(4-tert-butoxycarbonyl-(2-fluoro-3-thiazolidinyl)-2-oxoethyl]ureido}phenylacetic acid,
- 2-{3-{3-[2-((2R,4R)-4-tert-butoxycarbonyl-2-(2-fluorophenyl)-3-thiazolidinyl)-2-oxoethyl]ureido}phenyl}propionic acid,
- potassium (RS)-1-{3-{3-[2-((2R,4R)-4-tert-butoxycarbonyl-2-phenyl-3-thiazolidinyl))-2-oxoethyl]-ureido}phenyl}ethanesulphonate,
- potassium (RS)-1-{3-{3-[2-((2S,5R)-2-tert-butoxycarbonyl-5-phenyl-1-pyrrolidinyl)-2-oxoethyl]-ureido}phenyl}ethanesulphonate,
- potassium (2S,5R)-1-{3-{3-[2-(2-tert-butoxycarbonyl-5-phenyl-1-pyrrolidinyl)-2-oxoethyl]ureido}phenylmethanesulphonate,
- (2S,5R)-3-{3-[2-2-tert-butoxycarbonyl-5-phenyl-1-pyrrolidinyl)-2-oxoethyl]ureido}benzoic acid,
- (2RS,5SR)-3-{3-{2-[2-tert-butoxycarbonyl-5-(2-fluorophenyl)-1-pyrrolidinyl]-2-oxoethyl}ureido}benzoic acid,
- (cis)-3-{3-[2-(2,5-diphenyl-1-pyrrolidinyl)-2-oxoethyl]ureido}benzoic acid,
- (2RS,5SR)-3-{{2-[2-(2-hydroxyphenyl)-5-phenyl-1-pyrrolidinyl]-2-oxoureido}phenylacetic acid,
- (2R,4R)-3-{3-[2-(4-tert-butoxycarbonyl-2-phenyl-3-thiazolidinyl)-2-oxoethyl]ureido}phenylacetic acid,
- (2R,4R)-3-{3-[2-(4-tert-butoxycarbonyl-2-phenyl-3-thiazolidinyl)-2-oxoethyl]ureido}benzoic acid,
- 2-{3-{3-[2-((lRS,2R,4R)-4-tert-butoxycarbonyl-2-(2-fluorophenyl)-1-oxide-3-thiazolidinyl)-2-oxoethyl}ureido}phenyl}propionic acid,
- (2R,4R)-3-{3-[2-(4-tert-butoxycarbonyl-2-(2,3-difluorophenyl)-3-thiazolidinyl)-2-oxoethyl]ureido}phenylacetic acid,
- tert-butyl (2RS,5SR)-1-{2-{3-[(E)-3-(1-(hydroxyimino)ethyl)phenyl]ureido}acetyl}-5-phenylprolinate,
their racemates and enantiomers, when they contain at least one asymmetric carbon, and their salts to the preparation of a medicament intended for the treatment of chronic alcoholism or of conditions due to alcohol abuse.

4. Application of 2-{3-{3-[2-((2R,4R)-4-tert-butoxycarbonyl-2-(2-fluorophenyl)-3-thiazolidinyl)-2-oxoethyl]ureido}phenyl}propionic acid to the preparation of a medicament intended for the treatment of chronic alcoholism or of conditions due to alcohol abuse.

5. Application of the compounds of formula: 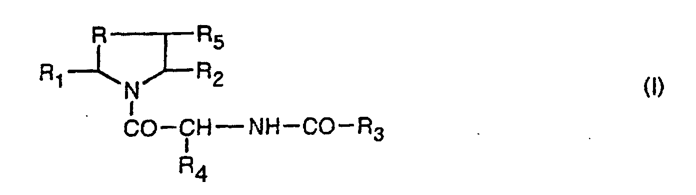 in which
- either R represents a methylene, ethylene, SO, SO₂, or CHOH radical or a sulphur atom, R₁ represents a pyridyl radical which is optionally substituted by one or more alkyl radicals, a furyl radical which is optionally substituted by one or more alkyl radicals, a thienyl radical which is optionally substituted by one or more alkyl radicals, a quinolyl radical which is optionally substituted by one or more alkyl radicals, a naphthyl radical which is optionally substituted by one or more alkyl radicals, an indolyl radical which is optionally substituted by one or more alkyl radicals or a phenyl radical which is optionally substituted by one or more substituents chosen from halogen atoms and alkyl, alkoxy, hydroxyl, nitro, amino, monoalkylamino, dialkylamino, alkoxycarbonyl, -CO-NR₇R₈, -NH-CO-CH₃, trifluoromethyl or trifluoromethoxy radicals and R₅ represents a hydrogen atom,
- or R represents a methylene radical, R₁ represents a hydrogen atom and R₅ represents a phenyl radical,
- or R represents a CHR₆ radical and R₁ and R₅ each represent a hydrogen atom,
- R₂ represents an alkoxycarbonyl, cycloalkyloxycarbonyl, cycloalkylalkyloxycarbonyl, -CONR₉R₁₀ or phenyl radical which is optionally substituted by one or more substituents chosen from alkyl, alkoxy or hydroxyl radicals,
- R₃ represents a phenyl radical (optionally substituted by one or more substituents chosen from halogen atoms and alkyl, alkoxy and alkylthio radicals), a naphthyl radical, an indolyl radical, a quinolyl radical or a phenylamino radical in which the phenyl ring is optionally substituted by one or more substituents chosen from halogen atoms and alkyl, alkoxy, alkylthio, trifluoromethyl, carboxyl, alkoxycarbonyl, hydroxyl, nitro, amino, acyl, cyano, sulphamoyl, carbamoyl, hydroxyiminoalkyl, alkoxyiminoalkyl, hydroxyaminocarbonyl, alkoxyaminocarbonyl, tetrazol-5-yl, tetrazol-5-ylalkyl, trifluoromethylsulphonamido, alkylsulphinyl, mono- or polyhydroxyalkyl, sulpho, -alk-O-CO-alk, -alk-COOX, -alk-O-alk, -alk'-COOX, -O-alk-COOX, -CH=CH-COOX, -CO-COOX, -alk-SO₃H in the salt form, -CH=CH-alk', - C(=NOH)-COOX, -S-alk-COOX, -O-CH₂-alk'-COOX, -CX=N-O-alk-COOX, -alk-N(OH)-CO-alk or 2,2-dimethyl-4,6-dioxo-1,3-dioxan-5-yl radicals,
- R₄ represents a hydrogen atom or an alkyl radical,
- R₆ represents a phenyl radical,
- R₇ represents a hydrogen atom or an alkyl, phenylalkyl or phenyl radical which is optionally substituted by one or more substituents chosen from halogen atoms and alkyl, alkoxy and alkylthio radicals,
- R₈ represents an alkyl, phenylalkyl or phenyl radical which is optionally substituted by one or more substituents chosen from halogen atoms and alkyl, alkoxy and alkylthio radicals,
or else R₇ and R₈ form, with the nitrogen atom to which they are attached, a saturated or unsaturated, mono- or polycyclic heterocycle containing 4 to 9 carbon atoms and one or more heteroatoms (O, N) and which is optionally substituted by one or more alkyl radicals,
- R₉ represents a hydrogen atom or an alkyl, cycloalkylalkyl, cycloalkyl, phenylalkyl or phenyl radical which is optionally substituted by one or more substituents chosen from halogen atoms and alkyl, alkoxy and alkylthio radicals,
- R₁₀ represents an alkyl, cycloalkylalkyl, cycloalkyl, phenylalkyl or phenyl radical which is optionally substituted by one or more substituents chosen from halogen atoms and alkyl, alkoxy and alkylthio radicals,
or else R₉ and R₁₀ form, together with the nitrogen atom to which they are attached, a saturated or unsaturated, mono- or polycyclic heterocycle containing 4 to 9 carbon atoms and one or more heteroatoms (O, N, S) and which is optionally substituted by one or more alkyl radicals,
- X represents a hydrogen atom or an alkyl or phenylalkyl radical,
- alk represents an alkyl or alkylene radical,
- alk' represents a hydroxyalkyl, hydroxyalkylene, alkoxyalkyl or alkoxyalkylene radical, it being understood that the alkyl, alkylene and alkoxy radicals and the alkyl, alkylene and alkoxy portions contain 1 to 4 straight- or branched-chain carbon atoms, the acyl radicals and portions contain 2 to 4 carbon atoms and the cycloalkyl radicals and portions contain 3 to 6 carbon atoms, and their salts and their racemates and enantiomers, when they contain at least one asymmetric centre, to the preparation of a medicament which allows the patient of his own accord to reduce his consumption of alcohol.

6. Application of a compound of formula (I) according to Claim 5 for which R represents a methylene radical, a sulphur atom or an SO radical, R₁ represents an optionally substituted phenyl radical, R₂ represents a phenyl or alkoxycarbonyl radical, R₄ and R₅ represent a hydrogen atom and R₃ represents a phenylamino radical in which the phenyl ring is substituted by a carboxyl, -alk-COOH, -S-alk-COOH, hydroxyalkyl, alk'-COOH or alk-SO₃H radical in the salt form to the preparation of a medicament which allows the patient of his own accord to reduce his consumption of alcohol.

7. Application of a compound of formula (I) according to Claim 5 chosen from the following compounds:
- tert-butyl (2RS,5SR)-1-{2-[3-(3-(RS)-1-hydroxyethyl)-phenyl)ureido]acetyl}-5-phenylprolinate,
- 2-{3-{3-[2-((2S,5R)-2-tert-butoxycarbonyl-5-phenyl-1-pyrrolidinyl)-2-oxoethyl]ureido}phenyl}propionic acid
- (2RS,5SR)-{3-{3-[2-(2-tert-butoxycarbonyl-5-phenyl-1-pyrrolidinyl)-2-oxoethyl]ureido}phenylthio}acetic acid,
- (2R,4R)-3-{3-[2-(4-tert-butoxycarbonyl-(2-fluoro-3-thiazolidinyl)-2-oxoethyl]ureido}phenylacetic acid,
- 2-{3-{3-[2-((2R,4R)-4-tert-butoxycarbonyl-2-(2-fluorophenyl)-3-thiazolidinyl)-2-oxoethyl]ureido}phenyl}propionic acid,
- potassium (RS)-1-{3-{3-[2-((2R,4R)-4-tert-butoxycarbonyl-2-phenyl-3-thiazolidinyl))-2-oxoethyl]-ureido}phenyl}ethanesulphonate,
- potassium (RS)-1-{3-{3-[2-((2S,5R)-2-tert-butoxycarbonyl-5-phenyl-1-pyrrolidinyl)-2-oxoethyl]-ureido}phenyl}ethanesulphonate,
- potassium (2S,5R)-1-{3-{3-[2.-(2-tert-butoxycarbonyl-5-phenyl-1-pyrrolidinyl)-2-oxoethyl]ureido}phenylmethanesulphonate,
- (2S,5R)-3-{3-[2-2-tert-butoxycarbonyl-5-phenyl-1-pyrrolidinyl)-2-oxoethyl]ureido}benzoic acid,
- (2RS,5SR)-3-{3-{2-[2-tert-butoxycarbonyl-5-(2-fluorophenyl)-1-pyrrolidinyl]-2-oxoethyl}ureido}benzoic acid,
- (cis)-3-(3-[2-(2,5-diphenyl-1-pyrrolidinyl)-2-oxoethyl]ureido}benzoic acid,
- (2RS,5SR)-3-{{2-[2-(2-hydroxyphenyl)-5-phenyl-1-pyrrolidinyl]-2-oxoureido}phenylacetic acid,
- (2R,4R)-3-{3-[2-(4-tert-butoxycarbonyl-2-phenyl-3-thiazolidinyl)-2-oxoethyl]ureido}phenylacetic acid,
- (2R,4R)-3-{3-[2-(4-tert-butoxycarbonyl-2-phenyl-3-thiazolidinyl)-2-oxoethyl]ureido}benzoic acid,
- 2-{3-{3-[2-((1RS,2R,4R)-4-tert-butoxycarbonyl-2-(2-fluorophenyl)-1-oxide-3-thiazolidinyl)-2-oxoethyl}ureido}phenyl}propionic acid,
- (2R,4R)-3-{3-[2-(4-tert-butoxycarbonyl-2-(2,3-difluorophenyl)-3-thiazolidinyl)-2-oxoethyl]ureido)-phenylacetic acid,
- tert-butyl (2RS,5SR)-1-{2-{3-[(E)-3-(1-(hydroxyimino)ethyl)phenyl]ureido}acetyl}-5-phenylprolinate,
their racemates and enantiomers, when they contain at least one asymmetric carbon, and their salts to the preparation of a medicament which allows the patient of his own accord to reduce his consumption of alcohol.

8. Application of 2-{3-{3-[2-((2R,4R)-4-tert-butoxycarbonyl-2-(2-fluorophenyl)-3-thiazolidinyl)-2-oxoethyl]ureido}phenyl}propionic acid to the preparation of a medicament which allows the patient of his own accord to reduce his consumption of alcohol.

## Patentansprüche

1. Anwendung der Verbindungen der Formel: 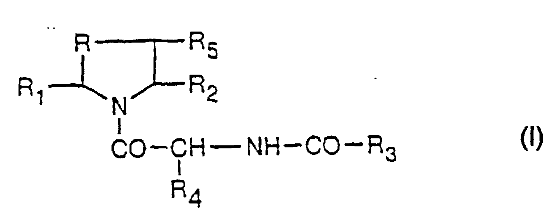 worin:
- R entweder einen Rest Methylen, Ethylen, SO, SO₂, CHOH oder ein Schwefelatom darstellt, R₁ darstellt einen Rest Pyridyl, gegebenenfalls substituiert mit einem oder mehreren Alkylresten, Furyl, gegebenenfalls substituiert mit einem oder mehreren Alkylresten, Thienyl, gegebenenfalls substituiert mit einem oder mehreren Alkylresten, Chinolyl, gegebenenfalls substituiert mit einem oder mehreren Alkylresten, Naphthyl, gegebenenfalls substituiert mit einem oder mehreren Alkylresten, Indolyl, gegebenenfalls substituiert mit einem oder mehreren Alkylresten, oder Phenyl, gegebenenfalls substituiert mit einem oder mehreren Substituenten, die ausgewählt sind unter den Halogenatomen und den Resten Alkyl, Alkoxy, Hydroxy, Nitro, Amino, Monoalkylamino, Dialkylamino, Alkoxycarbonyl, -CO-NR₇R₈, -NH-CO-CH₃, Trifluormethyl oder Trifluormethoxy, und R₅ ein Wasserstoffatom darstellt,
- oder R einen Methylenrest darstellt, R₁ ein Wasserstoffatom darstellt und R₅ einen Phenylrest darstellt,
- oder R einen Rest CHR₆ darstellt, R₁ und R₅ jeweils ein Wasserstoffatom darstellen,
- R₂ darstellt einen Rest Alkoxycarbonyl, Cycloalkyloxycarbonyl, Cycloalkylalkyloxycarbonyl, -CONR₉R₁₀ oder Phenyl, gegebenenfalls substituiert mit einem oder mehreren Substituenten, die ausgewählt sind unter den Resten Alkyl, Alkoxy oder Hydroxy,
- R₃ darstellt einen Rest Phenyl (gegebenenfalls substituiert mit einem oder mehreren Substituenten, die ausgewählt sind unter den Halogenatomen und den Resten Alkyl, Alkoxy und Alkylthio), Naphthyl, Indolyl, Chinolyl oder Phenylamino, wobei der Phenylkern gegebenenfalls substituiert ist mit einem oder mehreren Substituenten, die ausgewählt sind unter den Halogenatomen und den Resten Alkyl, Alkoxy, Alkylthio, Trifluormethyl, Carboxy, Alkoxycarbonyl, Hydroxy, Nitro, Amino, Acyl, Cyano, Sulfamoyl, Carbamoyl, Hydroxyiminoalkyl, Alkoxyiminoalkyl, Hydroxyaminocarbonyl, Alkoxyaminocarbonyl, 5-Tetrazolyl, 5-Tetrazolylalkyl, Trifluormethylsulfonamido, Alkylsulfinyl, Mono- oder Polyhydroxyalkyl, Sulfo, -alk-O-CO-alk, -alk-COOX, -alk-O-alk, -alk'-COOX, -O-alk-COOX, -CH=CH-COOX, -CO-COOX, -alk-SO₃H in Form eines Salzes, -CH=CH-alk', -C(=NOH)-COOX, -S-alk-COOX, -O-CH₂-alk'-COOX, -CX=N-O-alk-COOX, -alk-N(OH)-CO-alk oder 2,2-Dimethyl-4,6-dioxo-1,3-dioxan-5-yl,
- R₄ ein Wasserstoffatom oder einen Alkylrest darstellt,
- R₆ einen Phenylrest darstellt,
- R₇ darstellt ein Wasserstoffatom oder einen Rest Alkyl, Phenylalkyl oder Phenyl, gegebenenfalls substituiert mit einem oder mehreren Substituenten, die ausgewählt sind unter den Halogenatomen und den Resten Alkyl, Alkoxy und Alkylthio,
- R₈ darstellt einen Rest Alkyl, Phenylalkyl oder Phenyl, gegebenenfalls substituiert mit einem oder mehreren Substituenten, die ausgewählt sind unter den Halogenatomen und den Resten Alkyl, Alkoxy und Alkylthio,
oder aber R₇ und R₈ mit dem Stickstoffatom, an das sie gebunden sind, einen gesättigten oder ungesättigten mono- oder polycyclischen Heterocyclus mit 4 bis 9 Kohlenstoffatomen und einem oder mehreren Heteroatomen (O, N), der gegebenenfalls mit einem oder mehreren Alkylresten substituiert ist, bilden,
- R₉ darstellt ein Wasserstoffatom oder einen Rest Alkyl, Cycloalkylalkyl, Cycloalkyl, Phenylalkyl oder Phenyl, gegebenenfalls substituiert mit einem oder mehreren Substituenten, die ausgewählt sind unter den Halogenatomen und den Resten Alkyl, Alkoxy und Alkylthio,
- R₁₀ darstellt einen Rest Alkyl, Cycloalkylalkyl, Cycloalkyl, Phenylalkyl oder Phenyl, gegebenenfalls substituiert mit einem oder mehreren Substituenten, die ausgewählt sind unter den Halogenatomen und den Resten Alkyl, Alkoxy und Alkylthio,
oder aber R₉ und R₁₀ zusammen mit dem Stickstoffatom, an das sie gebunden sind, einen gesättigten oder ungesättigten mono- oder polycyclischen Heterocyclus mit 4 bis 9 Kohlenstoffatomen und einem oder mehreren Heteroatomen (O, N, S), der gegebenenfalls mit einem oder mehreren Alkylresten substituiert ist, bilden,
- X ein Wasserstoffatom, einen Alkyl- oder Phenylalkylrest darstellt,
- alk einen Alkyl- oder Alkylenrest darstellt,
- alk' einen Hydroxyalkyl-, Hydroxyalkylen-, Alkoxyalkyl- oder Alkoxyalkylenrest darstellt, wobei es sich versteht, dass die Alkyl-, Alkylen- und Alkoxyreste und die Alkyl-, Alkylen- und Alkoxyteile 1 bis 4 Kohlenstoffatome in gerader oder verzweigter Kette enthalten, die Acylreste und -teile 2 bis 4 Kohlenstoffatome enthalten und die Cyclalkylreste und -teile 3 bis 6 Kohlenstoffatome enthalten, sowie ihrer Salze und ihrer Racemate und Enantiomere, wenn sie wenigstens ein asymmetrisches Zentrum umfassen, zur Herstellung eines Medikaments, das für die Behandlung des chronischen Alkoholismus oder der Zustände aufgrund von Alkoholmissbrauch bestimmt ist.

2. Anwendung einer Verbindung der Formel (I) gemäß Anspruch 1, für die R einen Methylenrest, ein Schwefelatom oder einen Rest SO darstellt, R₁ einen gegebenenfalls substituierten Phenylrest darstellt, R₂ einen Phenyl- oder Alkoxycarbonylrest darstellt, R₄ und R₅ ein Wasserstoffatom darstellen und R₃ einen Phenylaminorest darstellt, dessen Phenylkern substituiert ist mit einem Rest Carboxy, -alk-COOH, -S-alk-COOH, Hydroxyalkyl, alk'-COOH oder alk-SO₃H in Form eines Salzes, zur Herstellung eines Medikaments, das für die Behandlung des chronischen Alkoholismus oder der Zustände aufgrund von Alkoholmissbrauch bestimmt ist.

3. Anwendung einer Verbindung der Formel (I) gemäß Anspruch 1, die ausgewählt ist unter den folgenden Verbindungen:
- (2RS,5SR)-1-{2-[3-(3-((RS)-1-Hydroxyethyl)phenyl)ureido]acetyl}-5-phenyl-prolinsäure-tert-butylester,
- 2-{3-{3-[2-((2S,5R)-2-tert-Butoxycarbonyl-5-phenyl-1-pyrrolidinyl)-2-oxoethyl]-ureido}phenyl}propionsäure,
- (2RS,5SR)-{3-{3-[2-(2-tert-Butoxycarbonyl-5-phenyl-1-pyrrolidinyl)-2-oxoethyl]-ureido}phenylthio}essigsäure,
- (2R,4R)-3-{3-[2-(4-tert-Butoxycarbonyl-2-(2-fluorphenyl)-3-thiazolidinyl)-2-oxoethyl]ureido}phenylessigsäure,
- 2-{3-{3-[2-((2R,4R)-4-tert-Butoxycarbonyl-2-(2-fluorphenyl)-3-thiazolidinyl)-2-oxoethyt]ureido}phenyl}propionsäure,
- (R,S)-1-{3-{3-[2-((2R,4R)-4-tert-Butoxycarbonyl-2-phenyl-3-thiazolidinyl)-2-oxoethyl]ureido}phenyl}ethansulfonat Kaliumsalz,
- (R,S)-1-{3-{3-[2-((2S,5R)-2-tert-Butoxycarbonyl-5-phenyl-1-pyrrolidinyl)-2-oxoethyl]ureido}phenyl}ethansulfonat Kaliumsalz,
- (2S,5R)-1-{3-{3-[2-(2-tert-Butoxycarbonyl-5-phenyl-1-pyrrolidinyl)-2-oxoethyl]-ureido}phenyl}methansulfonat Kaliumsalz,
- (2S,5R)-3-{3-[2-(2-tert-Butoxycarbonyl-5-phenyl-1-pyrrolidinyl)-2-oxoethyl]ureido}benzoesäure,
- (2RS,5SR)-3-{3-{2-[2-tert-Butoxycarbonyl-5-(2-fluorphenyl)-1-pyrrolidinyl]-2-oxoethyl}ureido}benzoesäure,
- cis-3-{3-[2-(2,5-Diphenyl-1-pyrrolidinyl)-2-oxoethyl]ureido}benzoesäure,
- (2RS,5SR)-3-{3-{2-[2-(2-Hydroxyphenyl)-5-phenyl-1-pyrrolidinyl]-2-oxoethyl}ureido}phenylessigsäure,
- (2R,4R)-3-{3-[2-(4-tert-Butoxycarbonyl-2-phenyl-3-thiazolidinyl)-2-oxoethyl]ureido}phenylessigsäure,
- (2R,4R)-3-{3-[2-(4-tert-Butoxycarbonyl-2-phenyl-3-thiazolidinyl)-2-oxoethyl]ureido}benzoesäure,
- 2-{3-{3-[2-((1RS,2R,4R)-4-tert-Butoxycarbonyl-2-(2-fluorphenyl)-1-oxid-3-thiazolidinyl)-2-oxoethyl]ureido}phenyl}propionsäure,
- (2R,4R)-3-{3-[2-(4-tert-Butoxycarbonyl-2-(2,3-difluorphenyl)-3-thiazolidinyl)-2-oxoethyl]ureido}phenylessigsäure,
- (2RS,5SR)-1-{2-{3-[(E)-3-(1-Hydroxyiminoethyl)phenyl]ureido}acetyl}-5-phenylprolinsäure-tert-butylester,
ihrer Racemate und Enantiomere, wenn sie wenigstens einen asymmetrischen Kohlenstoff enthalten, und ihrer Salze zur Herstellung eines Medikaments, das für die Behandlung des chronischen Alkoholismus oder der Zustände aufgrund von Alkoholmissbrauch bestimmt ist.

4. Anwendung der 2-{3-{3-[2-((2R,4R)-4-tert-Butoxycarbonyl-2-(2-fluorphenyl)-3-thiazolidinyl)-2-oxoethyl]ureido}phenyl}propionsäure zur Herstellung eines Medikaments, das für die Behandlung des chronischen Alkoholismus oder der Zustände aufgrund von Alkoholmissbrauch bestimmt ist.

5. Anwendung der Verbindungen der Formel: 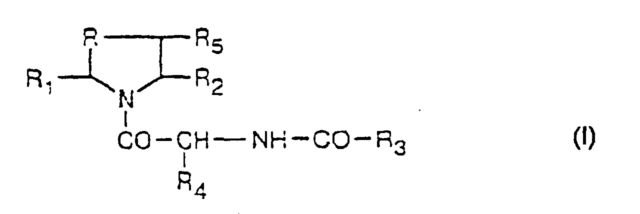 worin:
- R entweder einen Rest Methylen, Ethylen, SO, SO₂, CHOH oder ein Schwefelatom darstellt, R₁ darstellt einen Rest Pyridyl, gegebenenfalls substituiert mit einem oder mehreren Alkylresten, Furyl, gegebenenfalls substituiert mit einem oder mehreren Alkylresten, Thienyl, gegebenenfalls substituiert mit einem oder mehreren Alkylresten, Chinolyl, gegebenenfalls substituiert mit einem oder mehreren Alkylresten, Naphthyl, gegebenenfalls substituiert mit einem oder mehreren Alkylresten, Indolyl, gegebenenfalls substituiert mit einem oder mehreren Alkylresten, oder Phenyl, gegebenenfalls substituiert mit einem oder mehreren Substituenten, die ausgewählt sind unter den Halogenatomen und den Resten Alkyl, Alkoxy, Hydroxy, Nitro, Amino, Monoalkylamino, Dialkylamino, Alkoxycarbonyl, -CO-NR₇R₈, -NH-CO-CH₃, Trifluormethyl oder Trifluormethoxy, und R₅ ein Wasserstoffatom darstellt,
- oder R einen Methylenrest darstellt, R₁ ein Wasserstoffatom darstellt und R₅ einen Phenylrest darstellt,
- oder R einen Rest CHR₆ darstellt, R₁ und R₅ jeweils ein Wasserstoffatom darstellen,
- R₂ darstellt einen Rest Alkoxycarbonyl, Cycloalkyloxycarbonyl, Cycloalkylalkyloxycarbonyl, -CONR₉R₁₀ oder Phenyl, gegebenenfalls substituiert mit einem oder mehreren Substituenten, die ausgewählt sind unter den Resten Alkyl, Alkoxy oder Hydroxy,
- R₃ darstellt einen Rest Phenyl (gegebenenfalls substituiert mit einem oder mehreren Substituenten, die ausgewählt sind unter den Halogenatomen und den Resten Alkyl, Alkoxy und Alkylthio), Naphthyl, Indolyl, Chinolyl oder Phenylamino, wobei der Phenylkern gegebenenfalls substituiert ist mit einem oder mehreren Substituenten, die ausgewählt sind unter den Halogenatomen und den Resten Alkyl, Alkoxy, Alkylthio, Trifluormethyl, Carboxy, Alkoxycarbonyl, Hydroxy, Nitro, Amino, Acyl, Cyano, Sulfamoyl, Carbamoyl, Hydroxyiminoalkyl, Alkoxyiminoalkyl, Hydroxyaminocarbonyl, Alkoxyaminocarbonyl, 5-Tetrazolyl, 5-Tetrazolylalkyl, Trifluormethylsulfonamido, Alkylsu)finyl, Mono- oder Polyhydroxyalkyl, Sulfo, -alk-O-CO-alk. -alk-COOX, -alk-O-alk, -alk'-COOX, -O-alk-COOX, -CH=CH-COOX, -CO-COOX, -alk-SO₃H in Form eines Salzes, -CH=CH-alk', -C(=NOH)-COOX, -S-alk-COOX, -O-CH₂-alk'-COOX, -CX=N-O-alk-COOX, -alk-N(OH)-CO-alk oder 2,2-Dimethyl-4,6-dioxo-1,3-dioxan-5-yl,
- R₄ ein Wasserstoffatom oder einen Alkylrest darstellt,
- R₆ einen Phenylrest darstellt,
- R₇ darstellt ein Wasserstoffatom oder einen Rest Alkyl, Phenylalkyl oder Phenyl, gegebenenfalls substituiert mit einem oder mehreren Substituenten, die ausgewählt sind unter den Halogenatomen und den Resten Alkyl, Alkoxy und Alkylthio,
- R₈ darstellt einen Rest Alkyl, Phenylalkyl oder Phenyl, gegebenenfalls substituiert mit einem oder mehreren Substituenten, die ausgewählt sind unter den Halogenatomen und den Resten Alkyl, Alkoxy und Alkylthio,
oder aber R₇ und R₈ mit dem Stickstoffatom, an das sie gebunden sind, einen gesättigten oder ungesättigten mono- oder polycyclischen Heterocyclus mit 4 bis 9 Kohlenstoffatomen und einem oder mehreren Heteroatomen (O, N), der gegebenenfalls mit einem oder mehreren Alkylresten substituiert ist, bilden,
- R₉ darstellt ein Wasserstoffatom oder einen Rest Alkyl, Cycloalkylalkyl, Cycloalkyl, Phenylalkyl oder Phenyl, gegebenenfalls substituiert mit einem oder mehreren Substituenten, die ausgewählt sind unter den Halogenatomen und den Resten Alkyl, Alkoxy und Alkylthio,
- R₁₀ darstellt einen Rest Alkyl, Cycloalkylalkyl, Cycloalkyl, Phenylalkyl oder Phenyl, gegebenenfalls substituiert mit einem oder mehreren Substituenten, die ausgewählt sind unter den Halogenatomen und den Resten Alkyl, Alkoxy und Alkylthio,
oder aber R₉ und R₁₀ zusammen mit dem Stickstoffatom, an das sie gebunden sind, einen gesättigten oder ungesättigten mono- oder polycyclischen Heterocyclus mit 4 bis 9 Kohlenstoffatomen und einem oder mehreren Heteroatomen (O, N, S), der gegebenenfalls mit einem oder mehreren Alkylresten substituiert ist, bilden,
- X ein Wasserstoffatom, einen Alkyl- oder Phenylalkylrest darstellt,
- alk einen Alkyl- oder Alkylenrest darstellt,
- alk' einen Hydroxyalkyl-, Hydroxyalkylen-, Alkoxyalkyl- oder Alkoxyalkylenrest darstellt, wobei es sich versteht, dass die Alkyl-, Alkylen- und Alkoxyreste und die Alkyl-, Alkylen- und Alkoxyteile 1 bis 4 Kohlenstoffatome in gerader oder verzweigter Kette enthalten, die Acylreste und -teile 2 bis 4 Kohlenstoffatome enthalten und die Cyclalkylreste und -teile 3 bis 6 Kohlenstoffatome enthalten, sowie ihrer Salze und ihrer Racemate und Enantiomere, wenn sie wenigstens ein asymmetrisches Zentrum umfassen, zur Herstellung eines Medikaments, das es dem Patienten ermöglicht, seinen Alkoholkonsum von sich aus zu verringern.

6. Anwendung einer Verbindung der Formel (I) gemäß Anspruch 5, für die R einen Methylenrest, ein Schwefelatom oder einen Rest SO darstellt, R₁ einen gegebenenfalls substituierten Phenylrest darstellt, R₂ einen Phenyl- oder Alkoxycarbonylrest darstellt, R₄ und R₅ ein Wasserstoffatom darstellen und R₃ einen Phenylaminorest darstellt, dessen Phenylkern substituiert ist mit einem Rest Carboxy, -alk-COOH, -S-alk-COOH, Hydroxyalkyl, alk'-COOH oder alk-SO₃H in Form eines Salzes, zur Herstellung eines Medikaments, das es dem Patienten ermöglicht, seinen Alkoholkonsum von sich aus zu verringern.

7. Anwendung einer Verbindung der Formel (I) gemäß Anspruch 5, die ausgewählt ist unter den folgenden Verbindungen:
- (2RS,5SR)-1-{2-[3-(3-((RS)-1-Hydroxyethyl)phenyl)ureido]acetyl}-5-phenyl-prolinsäure-tert-butylester,
- 2-{3-{3-[2-((2S,5R)-2-tert-Butoxycarbonyl-5-phenyl-1-pyrrolidinyl)-2-oxoethyl]-ureido}phenyl}propionsäure,
- (2RS,5SR)-{3-{3-[2-(2-tert-Butoxycarbonyl-5-phenyl-1-pyrrolidinyl)-2-oxoethyl]-ureido}phenylthio}essigsäure,
- (2R,4R)-3-{3-[2-(4-tert-Butoxycarbonyl-2-(2-fluorphenyl)-3-thiazolidinyl)-2-oxoethyl]ureido}phenylessigsäure,
- 2-{3-{3-[2-((2R,4R)-4-tert-Butoxycarbonyl-2-(2-fluorphenyl)-3-thiazolidinyl)-2-oxoethyl]ureido}phenyl}propionsäure,
- (R,S)-1-{3-{3-[2-((2R,4R)-4-tert-Butoxycarbonyl-2-phenyl-3-thiazolidinyl)-2-oxoethyl]ureido}phenyl}ethansulfonat Kaliumsalz,
- (R,S)-1-{3-{3-[2-((2S,5R)-2-tert-Butoxycarbonyl-5-phenyl-1-pyrrolidinyl)-2-oxoethyl]ureido}phenyl}ethansulfonat Kaliumsalz,
- (2S,5R)-1-{3-{3-[2-(2-tert-Butoxycarbonyl-5-phenyl-1-pyrrolidinyl)-2-oxoethyl]-ureido}phenyl}methansulfonat Kaliumsalz,
- (2S,5R)-3-{3-[2-(2-tert-Butoxycarbonyl-5-phenyl-1-pyrrolidinyl)-2-oxoethyl]ureido}benzoesäure,
- (2RS,5SR)-3-{3-{2-[2-tert-Butoxycarbonyl-5-(2-fluorphenyl)-1-pyrrolidinyl]-2-oxoethyl}ureido}benzoesäure,
- cis-3-{3-[2-(2,5-Diphenyl-1-pyrrolidinyl)-2-oxoethyl]ureido}benzoesäure,
- (2RS,5SR)-3-{3-{2-[2-(2-Hydroxyphenyl)-5-phenyl-1-pyrrolidinyl]-2-oxoethyl}ureido}phenylessigsäure,
- (2R,4R)-3-{3-[2-(4-tert-Butoxycarbonyl-2-phenyl-3-thiazolidinyl)-2-oxoethyl]ureido}phenylessigsäure,
- (2R,4R)-3-{3-[2-(4-tert-Butoxycarbonyl-2-phenyl-3-thiazolidinyl)-2-oxoethyl]ureido}benzoesäure,
- 2-{3-{3-[2-((1RS,2R,4R)-4-tert-Butoxycarbonyl-2-(2-fluorphenyl)-1-oxid-3-thiazolidinyl)-2-oxoethyl]ureido}phenyl}propionsäure,
- (2R,4R)-3-{3-[2-(4-tert-Butoxycarbonyl-2-(2,3-difluorphenyl)-3-thiazolidinyl)-2-oxoethyl]ureido}phenylessigsäure,
- (2RS,5SR)-1-{2-{3-[(E)-3-(1-Hydroxyiminoethyl)phenyl]ureido}acetyl}-5-phenylprolinsäure-tert-butylester,
ihrer Racemate und Enantiomere, wenn sie wenigstens einen asymmetrischen Kohlenstoff enthalten, und ihrer Salze zur Herstellung eines Medikaments, das es dem Patienten ermöglicht, seinen Alkoholkonsum von sich aus zu veringern.

8. Anwendung der 2-{3-{3-[2-((2R,4R)-4-tert-Butoxycarbonyl-2-(2-fluorphenyl)-3-thiazolidinyl)-2-oxoethyl]ureido}phenyl}propionsäure zur Herstellung eines Medikaments, das das es dem Patienten ermöglicht, seinen Alkoholkonsum von sich aus zu veringem.
